(19) Europäisches Patentamt
European Patent Office
Office européen des brevets

(11) Numéro de publication : **0 515 272 A1**

(12) # DEMANDE DE BREVET EUROPEEN

(21) Numéro de dépôt : **92401385.7**

(22) Date de dépôt : **21.05.92**

(51) Int. Cl.$^5$ : **C07D 327/10,** C07D 303/22, C07D 303/36, A61K 31/165

(30) Priorité : **23.05.91 FR 9106235**

(43) Date de publication de la demande : **25.11.92 Bulletin 92/48**

(84) Etats contractants désignés : **PT**

(71) Demandeur : **RHONE-POULENC RORER SA**
**20, avenue Raymond Aron**
**F-92160 Antony (FR)**

(72) Inventeur : **Radisson, Xavier**
**20 rue Saint Maximin**
**F-69003 Lyon (FR)**

(74) Mandataire : **Pilard, Jacques et al**
**RHONE-POULENC RORER S.A. Direction Brevets (t144) 20 avenue Raymond Aron**
**F-92165 Antony Cédex (FR)**

(54) **Sulfates chiraux, leur préparation et leur emploi dans la synthèse de produits pharmaceutiques.**

(57) Les isomères (R) et (S) du sulfate de chlorométhyl-1 éthylène, leur préparation et leur emploi en particulier dans la synthèse de (S)-β-bloquants adrénergiques ou de la L-carnitine ou des phospholipides.

EP 0 515 272 A1

La présente invention concerne de nouveaux sulfates chiraux, leur préparation et leur emploi dans la synthèse de produits optiquement actifs.

Plus particulièrement, l'invention concerne les isomères (R) et (S) du sulfate de chlorométhyl-1 éthylène, leur préparation et leur emploi.

Selon la présente invention, les isomères (R) et (S) du sulfate de chlorométhyl-1 éthylène sont obtenus à partir, respectivement, des isomères (R) et (S) du chloro-3 propanediol-1,2 en passant intermédiairement par les isomères (R) et (S) du sulfite de chlorométhyl-1 éthylène correspondants.

Généralement, le sulfite de chlorométhyl-1 éthylène (R) ou (S) est obtenu à partir du (R)- ou (S)-chloro-3-propanediol-1,2 selon les méthodes connues et en particulier selon le procédé décrit pour la préparation du sulfite de chlorométhyl-1 éthylène racémique par D.S. Breslow et H. Skolnik, "The Chemistry of Heterocyclic Compounds - Multi-Sulphur and Sulphur and Oxygen 5- and 6- Membered Heterocycles", 1966, Part I, p. 1 and Part II, p. 663 ou par H.F. Van Woerden, Chem. Rev., 63, 557 (1963). Il est particulièrement avantageux de faire réagir un excès de chlorure de thionyle sur le chloro-3-propanediol-1,2 chiral en opérant à une température voisine de 0°C.

Généralement, le sulfate de chlorométhyl-1 éthylène (R) ou (S) est obtenu par oxydation du sulfite de chlorométhyl-1 éthylène correspondant en particulier selon le procédé décrit dans le brevet français FR 2 628 423 c'est-à-dire au moyen d'un hypohalogénite (hypochlorite ou hypobromite) alcalin ou alcalino-terreux, de préférence l'hypochlorite de sodium, de potassium ou de calcium, en présence d'une quantité catalytique d'un dérivé du ruthénium choisi parmi, de préférence, l'oxyde de ruthénium (IV) ($RuO_2$) et le chlorure de ruthénium ($RuCl_3$).

Le procédé peut être mis en oeuvre en milieu aqueux ou hydroorganique biphasique.

Lorsque le procédé est mis en oeuvre en milieu hydroorganique biphasique, le solvant est généralement choisi parmi les hydrocarbures aliphatiques ou cycloaliphatiques, éventuellement halogénés, tel que l'hexane, le cyclohexane, le chlorure de méthylène, le chloroforme, le tétrachlorure de carbone ou le dichloroéthane, ou les esters tels que l'acétate de méthyle ou d'éthyle.

Généralement on utilise un excès d'hypohalogénite par rapport au sulfite mis en oeuvre. De préférence on utilise 1 à 2 moles d'hypohalogénite par mole de sulfite.

Généralement, on utilise une quantité catalytique de dérivé du ruthénium comprise entre $10^{-6}$ et $10^{-1}$ mole par mole de sulfite mis en oeuvre.

Il est bien entendu que, par la mise en oeuvre du procédé selon l'invention, l'isomère (R) du sulfate de chlorométhyl-1 éthylène est obtenu à partir du (R)-chloro-3-propanediol-1,2 en passant par l'isomère (R) du sulfite de chlorométhyl-1 éthylène et que le sulfate de chlorométhyl-1 éthylène (S) est obtenu à partir du (S)-chloro-3-propanediol-1,2 en passant intermédiairement par l'isomère (S) du sulfite de chlorométhyl-1 éthylène.

Les isomères (R) et (S) du sulfate de chlorométhyl-1 éthylène sont particulièrement utiles dans la synthèse de produits chiraux présentant des propriétés thérapeutiques tels que les β-bloquants adrénergiques chiraux qui se caractérisent par la présence d'une chaîne alkylamino-3 hydroxy-2 propoxy.

Dans la famille des β-bloquants adrénergiques tels que l'acébutolol, l'aténolol, le propranolol, le céliprolol, le métoprolol, le timolol, le nadolol, le penbutolol, le levobunolol ou le pindolol, il est connu que l'activité thérapeutique est due à l'isomère (S), l'isomère (R) étant, d'une manière générale, totalement inactif. Il est donc particulièrement intéressant de pouvoir préparer directement l'isomère (S) pratiquement exempt de l'isomère (R).

Selon l'invention, les β-bloquants adrénergiques sous forme (S) tels que définis précédemment peuvent être obtenus selon l'une des méthodes suivantes:

1) par action d'un phénol préalablement anionisé sur l'isomère (R) du sulfate de chlorométhyl-1 éthylène suivi de l'action d'une alkylamine sur l'époxyde obtenu selon le schéma suivant :

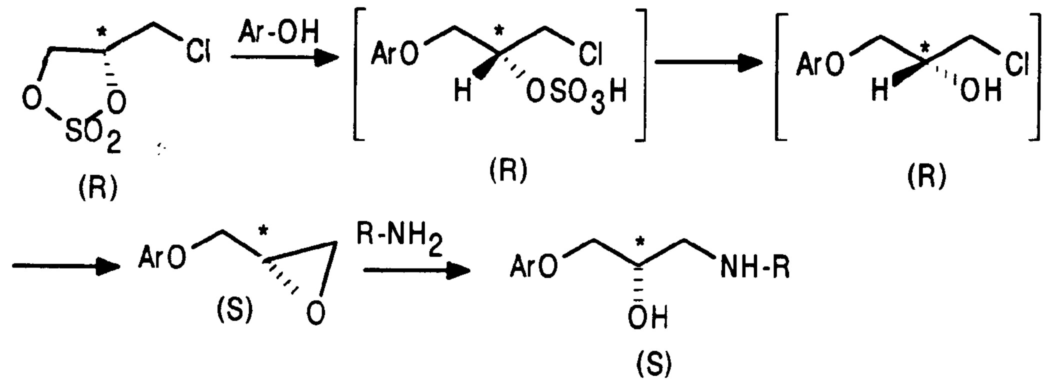


Dans ce schéma, Ar représente un radical aromatique ou un radical aromatique hétérocyclique éventuellement substitué que l'on rencontre dans les β-bloquants adrénergiques mentionnés ci-dessus et R représente un radical alcoyle droit ou ramifié contenant 1 à 4 atomes de carbone.

Généralement, la condensation de l'isomère (R) du sulfate de chlorométhyl-1 éthylène sur le phénol de formule ArOH, dans laquelle Ar est défini comme précédemment, est effectuée dans l'eau ou dans un solvant organique ou dans un milieu hydroorganique à une température comprise entre 0 et 80°C en présence d'une base choisie parmi les hydroxydes de métaux alcalins (soude), les carbonates ou bicarbonates de métaux alcalins (carbonate de sodium), l'ammoniaque ou les hydroxydes d'ammonium quaternaires (hydroxyde de tétrabutylammonium).

Comme solvants organiques peuvent être utilisés les nitriles (acétonitrile), les cétones (acétone), les alcools (éthanol), les esters (acétate d'éthyle), les amides (diméthylformamide) ou les hydrocarbures aliphatiques halogénés (chlorure de méthylène).

Généralement, on utilise un léger excès molaire, de préférence voisin de 10%, de sulfate cyclique par rapport au phénol.

Il n'est pas nécessaire d'isoler le sulfate et l'alcool chiral intermédiaires pour obtenir l'époxyde sous forme (S).

La condensation de l'amine de formule $R\text{-}NH_2$, dans laquelle R est défini comme précédemment, sur l'époxyde sous forme (S) est réalisée dans les conditions analogues à celles connues qui sont utilisées pour préparer les β-bloquants adrénergiques racémiques à partir de l'époxyde racémique.

2) par action d'une amine de formule $R\text{-}NH_2$ sur l'isomère S du sulfate de chlorométhyl-1 éthylène suivie de l'action d'un phénol Ar-OH préalablement anionisé selon le schéma suivant:

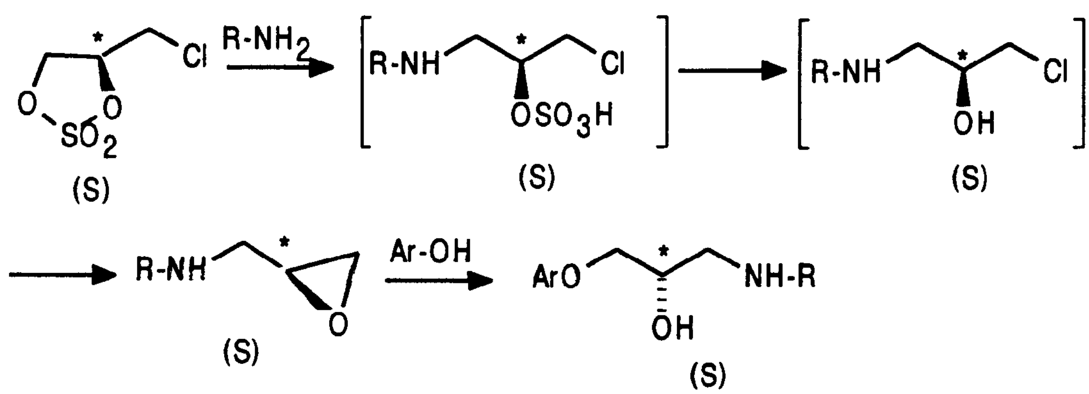


Dans ce schéma, Ar et R sont définis comme précédemment.

Généralement, la condensation de l'isomère (S) du sulfate de chlorométhyl-1 éthylène sur l'amine de formule $R\text{-}NH_2$, dans laquelle R est défini comme précédemment, est effectuée dans un solvant organique choisi, par exemple, parmi les hydrocarbures aliphatiques halogénés tels que le dichlorométhane à une température voisine de la température de reflux du mélange réactionnel.

Généralement, la condensation du phénol de formule ArOH, dans laquelle Ar est défini comme précédemment, sur l'époxyde de forme (S), obtenu après hydrolyse en milieu basique du produit de réaction de l'amine de formule $R\text{-}NH_2$ sur l'isomère (S) du sulfate de chlorométhyl-1 éthylène, est effectuée dans des conditions

identiques à celles décrites précédemment pour la condensation de l'isomère R du sulfate de chlorométhyl-1 éthylène sur le phénol de formule Ar-OH.

Le procédé de préparation des β-bloquants adrénergiques chiraux selon la présente invention présente, sur les procédés connus à partir de l'épichlorhydrine chirale, l'avantage d'être très sélectif, de mettre en oeuvre un sulfate cyclique chiral beaucoup plus réactif et de ne pas offrir de possibilité de racémisation.

Les isomères (R) et (S) du sulfate de chlorométhyl-1 éthylène peuvent également être avantageusement utilisés dans la synthèse d'autres produits chiraux tels que la L-carnitine, le "Platelet Activating Factor" ou divers (S)-phospholipides ou (S)-glycérolphosphates .

Les exemples suivants montrent comment l'invention peut être mise en pratique.

EXEMPLE 1

Dans un ballon tricol, on introduit 25 g de (R)-chloro-3-propanediol-1,2 (0,226 mole). Sous lente agitation, on ajoute en 1 heure, à 0°C, 26,9 g de chlorure de thionyle (0,226 mole). On observe un important dégagement gazeux ainsi qu'une fluidification du mélange réactionnel. Après la fin de l'addition, on laisse la température monter au voisinage de 25°C puis le mélange réactionnel est dégazé sous pression réduite puis distillé sous pression réduite (15 mm de mercure ; 2 kPa). On obtient ainsi 29,05 g de l'isomère (R) du sulfite de chlorométhyl-1 éthylène dont les caractéristiques sont les suivantes :

- P.E.$_{15}$ = 97-100°C
- ratio diastéréoisomérique: 37/63
- pouvoir rotatoire :

$[\alpha]^{27}_{D}$ = -37,5° (c = 10,45 x $10^{-3}$; chloroforme contenant 0,6 % d'éthanol)

$[\alpha]^{27}_{365}$ = -140,9° (c = 10,45 x $10^{-3}$ ; chloroforme contenant 0,6 % d'éthanol)

- pureté énantiomérique : supérieure à 99 %.

Le rendement est de 82, 1 %.

A une suspension de 10 g de l'isomère (R) du sulfite de chlorométhyl-1 éthylène (0,0639 mole) dans 80 $cm^3$ d'eau refroidie à 5°C, on ajoute en 40 minutes, 40 $cm^3$ d'une solution aqueuse d'hypochlorite de sodium 2,1M contenant 6 mg de $RuO_2$, $2H_2O$.

Le mélange réactionnel noircit tout au long de l'addition. A la fin de l'addition, on maintient pendant 5 minutes à 0°C puis on ajoute 50 $cm^3$ de chlorure de méthylène. Après décantation, la phase aqueuse est extraite 2 fois par 50 $cm^3$ de chlorure de méthylène. Les phases organiques réunies sont lavées par 50 $cm^3$ d'eau. Après séchage et filtration, le mélange réactionnel est concentré à sec à 50°C sous pression réduite (15 mm de mercure ; 2 kPa).

On obtient ainsi 8,85 g de l'isomère (R) du sulfate de chlorométhyl-1 éthylène dont les caractéristiques sont les suivantes :

- pouvoir rotatoire :

$[\alpha]^{27}_{D}$ = +13,5° (c = 17,58 x $10^{-3}$; chloroforme contenant 0,6 % d'éthanol) $[\alpha]^{27}_{365}$ = +38,2° (c = 17,58 x $10^{-3}$; chloroforme contenant 0,6 % d'éthanol)

- pureté énantiomérique : supérieure à 99 %
- pureté chimique : voisine de 97 %

Le rendement est de 80 % par rapport au sulfite mis en oeuvre.

EXEMPLE 2

Dans un ballon, on introduit 20 $cm^3$ d'acétone, 2,21 g d'acétyl-2 butyramido-4 phénol (10 mmoles), 2 g de l'isomère (R) du sulfate de chlorométhyl-1 éthylène (11,6 mmoles). On ajoute ensuite 0,54 g de soude en pastilles.

On agite pendant 3 heures à une température comprise entre 20 et 28°C jusqu'à l'obtention d'une solution homogène. On chauffe ensuite pendant 1 heure 30 minutes à la température de reflux du mélange réactionnel en présence de 0,8 g d'hydrogénosulfate de potassium et de 0,2 $cm^3$ d'acide sulfurique. Le précipité qui apparaît est séparé par filtration. Au filtrat, on ajoute 1,2 g de soude en pastilles. On maintient pendant 2 heures 30 minutes à une température voisine de 20°C. On reprend par de l'eau puis élimine l'acétone sous pression réduite (15 mm de mercure; 2 kPa). Le précipité obtenu est séparé par filtration, lavé à l'eau puis séché sous pression réduite.

On obtient ainsi 2, 17 g de (2S)-(acétyl-2'butyramide-4'phénoxy)-1 époxy-2,3 propane dont les caractéristiques sont les suivantes :

- P.F. = 139°C
- pouvoir rotatoire :

$[\alpha]^{27}_D$ = 13,9° (c = 3,88 x $10^{-3}$ ; chloroforme contenant 0,6 % d'éthanol)
$[\alpha]^{27}_{365}$ = 41,2° (c = 3,88 x $10^{-3}$ ; chloroforme contenant 0,6 % d'éthanol)
- pureté énantiomérique: supérieure à 99 %.
Le rendement est de 78,3 %.

## Revendications

**1 -** Les isomères (R) et (S) du sulfate de chlorométhyl-1 éthylène.

**2 -** Procédé de préparation des isomères (R) et (S) du sulfate de chlorométhyl-1 éthylène caractérisé en ce que l'on oxyde les isomères (R) et (S) du sulfite de chlorométhyl-1 éthylène au moyen d'un hypohalogénite alcalin ou alcalino-terreux en présence d'une quantité catalytique d'un dérivé du ruthénium en opérant en milieu aqueux ou hydroorganique à une température inférieure à 10°C.

**3 -** Procédé selon la revendication 2 caractérisé en ce que le dérivé du ruthénium est choisi parmi l'oxyde de ruthénium (IV) et le trichlorure de ruthénium.

**4 -** Procédé selon l'une des revendications 2 ou 3 caractérisé en ce que l'on utilise de $10^{-6}$ à $10^{-1}$ mole de dérivé du ruthénium par mole de sulfite.

**5 -** Procédé selon la revendication 2 caractérisé en ce que l'hypohalogénite alcalin ou alcalino-terreux est choisi parmi l'hypochlorite de sodium, l'hypochlorite de potassium et l'hypochlorite de calcium.

**6 -** Procédé selon l'une des revendications 2 ou 5 caractérisé en ce que l'on utilise 1 à 2 moles d'hypohalogénite par mole de sulfite.

**7 -** Procédé selon la revendication 2 caractérisé en ce que, lorsque l'on opère en milieu hydroorganique, le solvant et choisi parmi les hydrocarbures aliphatiques ou cycloaliphatiques éventuellement halogénés et les esters.

**8 -** Utilisation de l'isomère (R) du sulfate de chlorométhyl-1 éthylène pour la préparation de β-bloquants adrénergiques (S) de formule générale :

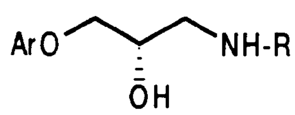

dans laquelle Ar représente un radical aromatique ou un radical aromatique hétérocyclique éventuellement substitué et R représente un radical alcoyle droit ou ramifié contenant 1 à 4 atomes de carbone, caractérisé en ce que l'on fait réagir l'isomère (R) du sulfate de chlorométhyl-1 éthylène sur un phénol de formule Ar-OH préalablement anionisé, en présence d'un excès de base, pour obtenir, après hydrolyse, un époxyde sous forme (S) de formule générale:

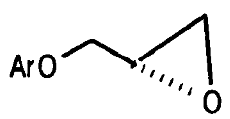

dans laquelle Ar est défini comme précédemment, sur lequel on fait réagir, selon les méthodes connues, une amine de formule $R-NH_2$ dans laquelle R est défini comme précédemment.

**9 -** Utilisation de l'isomère (S) du sulfate de chlorométhyl-1 éthylène pour la préparation de β-bloquants adrénergiques (S) de formule générale :

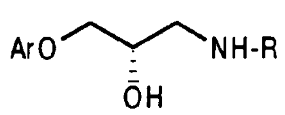

dans laquelle Ar et R sont définis comme dans la revendication 8 caractérisé en ce que l'on fait réagir l'isomère (S) du sulfate de chlorométhyl-1 éthylène sur une amine de formule $R-NH_2$, puis, après hydrolyse alcaline du mélange réactionnel, fait réagir un phénol de formule Ar-OH, préalablement anionisé, sur l'époxyde de formule générale :

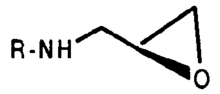

dans laquelle R est défini comme précédemment, ainsi obtenu.

**10 -** Utilisation des isomères (R) et (S) du sulfate de chlorométhyl-1 éthylène selon les revendications 8 et 9 pour la préparation de β-bloquants adrénergiques (S) de formule générale :

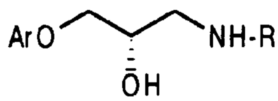

dans laquelle Ar représente le reste aromatique de l'acébutolol, de l'aténolol, du propranolol, ,du céliprolol, du métoprolol, du timolol, du nadolol, du penbutolol, du levobunolol ou du pindolol, et R représente un radical alcoyle droit ou ramifié contenant 1 à 4 atomes de carbone.

**11 -** Utilisation de l'isomère du sulfate de chlorométhyl-1 éthylène pour la préparation de la L-carnitine, de (S) -phospholipides et de (S)-glycérolphosphates.

Office européen des brevets

# RAPPORT DE RECHERCHE EUROPEENNE

Numero de la demande

EP 92 40 1385

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-0 332 521 (RHONE POULENC)<br>* page 1 - page 6 *<br>--- | 1-7 | C07D327/10<br>C07D303/22<br>C07D303/36<br>A61K31/165 |
| A | EP-A-0 399 899 (RHONE POULENC)<br>* page 1 - page 5 *<br>----- | 8-10 | |

| DOMAINES TECHNIQUES RECHERCHES (Int. Cl.5 ) |
|---|
| C07D |

Le présent rapport a été établi pour toutes les revendications

| Lieu de la recherche | Date d'achèvement de la recherche | Examinateur |
|---|---|---|
| LA HAYE | 17 AOUT 1992 | FRANCOIS J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)